(19) Europäisches Patentamt
European Patent Office
Office européen des brevets

(11) EP 1 055 414 B1

(12) EUROPÄISCHE PATENTSCHRIFT

(45) Veröffentlichungstag und Bekanntmachung des Hinweises auf die Patenterteilung:
**06.08.2003 Patentblatt 2003/32**

(51) Int Cl.[7]: **A61K 7/42**

(21) Anmeldenummer: **00109131.3**

(22) Anmeldetag: **05.05.2000**

(54) **Kosmetische oder dermatologische Lichtschutzzubereitungen mit einem Gehalt an Bornitrid und sulfonierten UV-Filtersubstanzen sowie die Verwendung von Bornitrid zur Stabilisierung von Emulsionen, die einen Gehalt an Elektrolyten, insbesondere einen Gehalt an sulfonierten UV-Filtersubstanzen, aufweisen**

Cosmetic or dermatological photoprotective preparations containing boron nitride and sulfonated UV filters as well as the use of boron nitride to stabilize emulsions containing electrolytes, especially sulfonated UV filters

Formulations cosmétiques ou dermatologiques de protection solaire contenant du nitrure de bore et des filtres UV sulfonés ainsi que l'utilisation de nitrure de bore pour stabiliser des émulsions contenant des électrolytes, plus spéciallement des filtres UV sulfonés

(84) Benannte Vertragsstaaten:
**AT BE CH CY DE DK ES FI FR GB GR IE IT LI LU MC NL PT SE**

(30) Priorität: **22.05.1999 DE 19923773**

(43) Veröffentlichungstag der Anmeldung:
**29.11.2000 Patentblatt 2000/48**

(73) Patentinhaber: **Beiersdorf Aktiengesellschaft
20245 Hamburg (DE)**

(72) Erfinder:
- **Gers-Barlag, Heinrich, Dr.
25495 Kummerfeld (DE)**
- **Müller, Anja
23843 Rümpel (DE)**

(56) Entgegenhaltungen:
**EP-A- 0 796 612 EP-A- 0 987 001
EP-A- 0 987 002 EP-A- 0 987 003
EP-A- 0 987 005 EP-A- 0 987 006
EP-A- 0 987 007 EP-A- 0 987 008
EP-A- 1 013 262 EP-A- 1 013 263
WO-A-00/07549 DE-A- 19 933 463**

- **CHEMICAL ABSTRACTS, vol. 129, no. 12, 12. September 1999 (1999-09-12) Columbus, Ohio, US; abstract no. 152981, INTROINI,CARLO: "Boron nitride as a component in creams and emulsions in general" Seite 878; XP002145869 & COSMET NEWS, Bd. 21, Nr. 119, 1998, Seiten 126-127,**
- **CHEMICAL ABSTRACTS, vol. 127, no. 10, 8. September 1997 (1997-09-08) Columbus, Ohio, US; abstract no. 140174, INTROINI,CARLO: "Boron nitride. A new ingredient in body and sun products" Seite 997; XP002145870 & COSMET TOILETRIES, ED. ITAL., Bd. 18, Nr. 2, 1997, Seiten 71-76,**

EP 1 055 414 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft kosmetische und dermatologische Formulierungen, insbesondere kosmetische und dermatologische Lichtschutzformulierungen, bevorzugt kosmetische und dermatologische Lichtschutzformulierungen, welche in Form von O/W-Emulsionen, vorliegen.

Die schädigende Wirkung des ultravioletten Teils der Sonnenstrahlung auf die Haut ist allgemein bekannt In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat daher keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. Als ein Maximum der Erythemwirksamkeit des Sonnenlichtes wird der engere Bereich um 308 nm angegeben.

Zum Schutz gegen UV-B-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Man hat lange Zeit fälschlicherweise angenommen, daß die langwellige UV-A-Strahlung mit einer Wellenlänge zwischen 320 nm und 400 nm nur eine vemachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u.a. erwiesen, daß selbst die UV-A-Strahlung unter ganz normalen Alltagsbedingungen ausreicht, um innerhalb kurzer Zeit die Collagen- und Elastinfasem zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Femer können bereits sehr geringe Strahlendosen photochemische Reaktionen auslösen. Hierzu gehört insbesondere die Bildung freier Radikale, welche wiederum aufgrund ihrer hohen Reaktivität unkontrollierte Folgereaktionen auslösen können. Um solchen Reaktionen vorzubeugen, können den kosmetischen bzw. dermatologischen Formulierungen zusätzlich Antioxidantien und/oder Radikalfänger zugesetzt werden. So ist beispielsweise vorgeschlagen worden, Vitamin E, eine Substanz mit bekannter antioxidativer Wirkung in Lichtschutzformulierungen einzusetzen, allerdings bleibt hier die erzielte Wirkung weit hinter der erhofften zurück.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z.B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Homschicht zurückgehalten werden.

Ein Sonnenbad wird von den meisten Menschen als angenehm empfunden, die nachteiligen Folgen zunächst nicht beachtet. Allerdings hat sich in den letzten Jahren durchaus ein Bewußtsein über die negativen Auswirkungen einer zu intensiven Sonnenbestrahlung herausgebildet, weshalb mehr und stärker schützende Sonnenschutzmittel angewendet werden.

Da die Beiträge der verschiedenen Wellenlängebereiche des UV-Lichtes zu lichtbedingten Hautveränderungen nicht vollständig geklärt sind, geht man heute verstärkt davon aus, daß vorbeugender Schutz sowohl gegen UV-A- als auch gegen UV-B-Strahlen, beispielsweise durch Auftrag von Lichtschutzfiltersubstanzen in Form einer kosmetischen oder dermatologischen Formulierung auf die Haut, von grundsätzlicher Wichtigkeit ist. Kosmetische oder dermatologische Mittel sollen, in dünner Schicht auf die Haut aufgetragen, diese vor den negativen Auswirkungen der Sonnenstrahlung schützen.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Je nachdem, in welchem Bereich des UV-Lichtes absorbiert wird, unterscheidet man UV-B-Filter, UV-A-Filter und Breitbandfilter (welche über den gesamten Bereich des UV-A und UV-B eine Filterwirkung zeigen). Durch entsprechende Auswahl des UV-Filters und seiner Konzentration im Sonnenschutzmittel hat man die Möglichkeit, den Grad der Abschirmung des UV-Lichtes zu beeinflussen. Für die Dosierung der Substanzen in den fertigen Formulierungen können die Extinktionswerte allerdings allenfalls eine Orientierungshilfe bieten, denn durch Wechselwirkungen mit Inhaltsstoffen der Formulierung oder der Haut selbst können Unwägbarkeiten auftreten. Femer ist in der Regel schwierig vorab abzuschätzen, wie gleichmäßig und in welcher Schichtdicke die Filtersubstanz in und auf der Homschicht der Haut verteilt ist.

Die Wirksamkeit von Sonnenschutzmittel bzw. der ihnen zugrunde liegenden UV-Filter wird in der Regel in biologischen Wirksamkeitsprüfungen unter standardisierten Bedingungen bestimmt.

Der Lichtschutzfaktor (LSF, oft auch SPF (sun protection factor) genannt) gibt die Verlängerung der Sonnenbestrahlung an, die durch Verwendung des Sonnenschutzmittels ermöglicht wird. Er ist der Quotient aus Erythemschwellenzeit mit Sonnenschutzmittel und Erythemschwellenzeit ohne Sonnenschutzmittel.

Zur Prüfung der UV-A-Schutzleistung wird üblicherweise die IPD-Methode verwendet (IPD ≡ immediate pigment darkening). Hierbei wird - ähnlich der Bestimmung des Lichtschutzfaktors - ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Eine andere, europaweit etablierte Prüfungsmethode ist der Australische Standard AS/NZS 2604:1997. Dabei wird die Absorption der Zubereitung im UV-A-Bereich gemessen. Um den Standard zu erfüllen, muß die Zubereitung mindestens 90 % der UV-A-Strahlung im Bereich 320 -360 nm absorbieren.

Die Einsatzkonzentration bekannter Lichtschutzfiltersubstanzen, insbesondere auch solcher, welche im UV-A-Bereich eine hohe Filterwirkung zeigen, ist häufig gerade in Kombination mit anderen als Festkörper vorliegenden Substanzen begrenzt Es bereitet daher gewisse formulierungstechnische Schwierigkeiten, höhere Lichtschutzfaktoren bzw. UV-A-Schutzleistung zu erzielen.

Das Patent Dokument EP-A-1 013 263 offenbart die Verwendung von Bornitrid zur Stabilisierung von W/O-Emulsionen, die einen Gehalt an Phenylen-1,4-bis-(2-benzimidazyl)-3,3-5,5-tetrasulfonsäure und/oder ihre Salze, enthalten.

Das Patent Dokument EP-A-0 987 001 offenbart die Verwendung von Bornitrid zur Stabilisierung von Emulsionen, die einen Gehalt an Phenylen-1,4-bis-(2-benzimidazyl)-3,3-5,5-tetrasulfonsäure und/oder ihre Salze, enthalten können.

Da Lichtschutzfiltersubstanzen in der Regel kostspielig sind und da manche Lichtschutzfiltersubstanzen zudem schwierig in höheren Konzentrationen in kosmetische oder dermatologische Zubereitungen einzuarbeiten sind, war es eine Aufgabe der Erfindung, auf einfache und preiswerte Weise zu Zubereitungen zu gelangen, welche bei ungewöhnlich niedrigen Konzentrationen an herkömmlichen UV-A-Lichtschutzfiltersubstanzen dennoch eine akzeptable oder sogar hohe UV-A-Schutzleistung erreichen.

Eine weitere Aufgabe der Erfindung war es, auf einfache und preiswerte Weise zu Zubereitungen zu gelangen, welche bei ungewöhnlich niedrigen Konzentrationen an herkömmlichen UV-B-Lichtschutzfiltersubstanzen dennoch akzeptable oder sogar hohe LSF-Werte erreichen.

Ferner war Aufgabe der Erfindung durch ungewöhnlich niedrige Konzentrationen an UV-A- und UV-B-Filtersubstanzen und/oder Breitbandfiltern eine dennoch akzeptable oder sogar hohe Breitbandwirkung zu erreichen.

Die vorliegende Erfindung betrifft eine Anwendung, welche es erlaubt, die Stabilität kosmetischer und dermatologischer Lichtschutzzubereitungen in Form von O/W-Emulsionen zu steigern.

Übliche kosmetische Darreichungsformen sind Emulsionen, also metastabile Zwei- oder Mehrphasensysteme bei welchen die einzelnen Phasen im flüssigen Zustande vorliegen. Die gängigsten Emulsionen sind O/W- und W/O-Emulsionen. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Um die Metastabilität von Emulsionen gewährleisten zu können, sind in der Regel grenzflächenaktive Substanzen, also Emulgatoren, nötig.

An sich ist die Verwendung der üblichen kosmetischen Emulgatoren völlig unbedenklich. Dennoch können Emulgatoren, wie letztlich jede chemische Substanz, im Einzelfalle allergische oder auf Überempfindlichkeit des Anwenders beruhende Reaktionen hervorrufen.

So ist bekannt, daß bei manchen besonders empfindlichen Personen bestimmte Lichtdermatosen durch gewisse Emulgatoren und gleichzeitige Einwirkung von Sonnenlicht ausgelöst werden.

Es ist möglich, emulgatorfreie Zubereitungen herzustellen, welche beispielsweise in einer wäßrigen Phase dispergierte Öltröpfchen, ähnlich einer O/W-Emulsion, aufweisen. Voraussetzung dafür kann sein, daß die kontinuierliche wäßrige Phase ein die dispergierte Phase stabilisierendes Gelgerüst aufweist und andere Umstände mehr. Solche Systeme werden gelegentlich Hydrodispersionen oder Oleodispersionen genannt, je nachdem, welches die disperse und welches die kontinuierliche Phase darstellt.

Es ist für die kosmetische Galenik aber weder nötig noch möglich, auf Emulgatoren ganz zu verzichten, zumal eine gewisse Auswahl an besonders milden Emulgatoren existiert. Allerdings besteht ein Mangel des Standes der Technik an einer befriedigend großen Vielfalt solcher Emulgatoren, welche dann auch das Anwendungsspektrum entsprechend milder und hautverträglicher kosmetischer Zubereitungen deutlich verbreitern würde.

So war eine Aufgabe der vorliegenden Erfindung, kosmetische bzw. dermatologische Zubereitungen mit hervorragenden hautpflegenden Eigenschaften zur Verfügung zu stellen.

Ein Nachteil insbesondere von O/W-Emulsionen ist oft deren mangelnde Stabilität gegenüber höheren Elektrolytkonzentrationen, was sich in Phasentrennung äußert. Dies kann zwar auch bei W/O-EMulsionen gelegentlich zu Problemen führen, tritt dort aber bei weitem nicht so in den Vordergrund wie bei O/W-Systemen. Zwar läßt sich diesen oft durch geeignete Wahl des Emulgatorsystems in gewissem Maße Abhilfe schaffen, es treten dann aber ebensooft andere Nachteile auf.

Es ist andererseits oft wünschenswert, bestimmte Elektrolyte einzusetzen, um deren sonstige physikalische, chemische bzw. physiologische Eigenschaften nutzen zu können.

Üblicherweise werden die Konzentrationen aller Bestandteile einer kosmetischen oder dermatologischen Zubereitungen in solchen Einheiten wie Gewichts-%, Mol-% und dergleichen angegeben. Aufgrund ihrer mehr oder minder stark ausgeprägten Dissoziation in Kationen und Anionen, oftmals in mehreren Dissoziationsstufen, erscheint es zweckmäßiger für die Schilderung der vorliegenden Erfindung und ihres technischen Hintergrundes, von der Ionenstärke eines gegebenen Elektrolytes in seiner Lösung auszugehen.

Die Ionenstärke *I* einer Elektrolytlösung ist definiert als

$$I = \frac{1}{2} \sum_i c_i \, z_i^2$$

wobei $c_i$ die Konzentrationen der einzelnen Ionensorten (in mol/l) und $z_i$ deren Ladungszahlen darstellen. Die physikalische Einheit der Ionenstärke ist die einer Konzentration (mol/l).

Eine 1-%ige ( = 0,17-molare) Kochsalzlösung hat beispielsweise eine Ionenstärke *I* = 0,17.

Bestimmte wasserlösliche, UV-Filtersubstanzen stellen Elektrolyte dar. Sie liegen zumeist als Alkalisalze bzw. in wäßriger Lösung in dissoziierter Form vor. Solche wasserlöslichen UV-Filtersubstanzen tragen zumeist an ihrem Molekülgerüst eine oder mehrere Sulfonsäuregruppen bzw. Sulfonatgruppen:

Die 2-Phenylbenzimidazol-5-sulfonsäure und ihre Salze, beispielsweise das Natrium-, Kalium- oder ihr Triethanolammonium-Salz

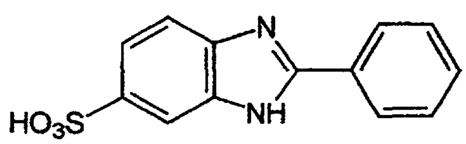

,

Sulfonsäure-Derivate von Benzophenonen, vorzugsweise 2-Hydroxy-4-methoxybenzophenon-5-sulfonsäure und ihre Salze, beispielsweise das entsprechende Natrium-, Kalium- oder Triethanolammonium-Salz:

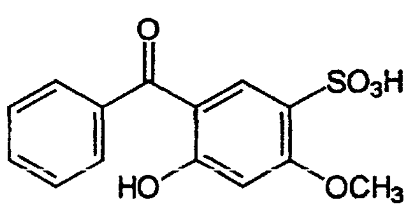

Sulfonsäure-Derivate des 3-Benzylidencamphers, wie z.B. 4-(2-Oxo-3-bornylidenmethyl)benzolsulfonsäure und ihre Salze, beispielsweise das entsprechende Natrium-, Kalium- oder Triethanolammonium-Salz:

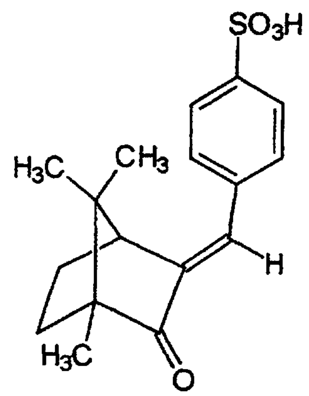

Die 2-Methyl-5-(2-oxo-3-bomylidenmethyl)benzolsulfonsäure und ihre Salze, beispielsweise das entsprechende Natrium-, Kalium- oder Triethanolammonium-Salz:

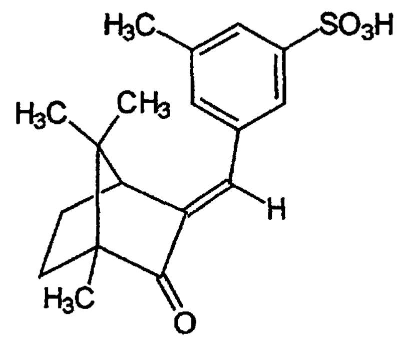

Das 1,4-di(2-oxo-10-Sulfo-3-bornylidenmethyl-)Benzol und dessen Salze (die entprehenden 10-Sulfato-verbindungen, beispielsweise das entsprechende Natrium-, Kaliumoder Triethanolammonium-Salz), auch als Benzol-1,4-di(2-oxo-3-bornylidenmethyl-10-Sulfonsäure bezeichnet

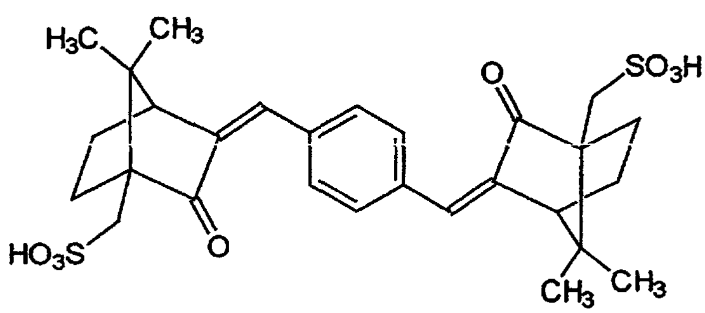

Für Emulsionen mit diesen UV-Filtersubstanzen gilt das gesagte, es ist im Einzelfalle schwierig, stabile Zubereitungen zu erstellen.

Eine weitere Aufgabe der vorliegenden Erfindung war es also, Lösungswege zu kosmetischen oder dermatologischen O/W-Emulsionen, aufzudecken, welche gegenüber erhöhten Elektrolytkonzentrationen - bzw. erhöhten Ionenstärken - stabil sind, wenn dies auf die Verwendung eines oder mehrerer wasserlöslicher UV-Filtersubstanzen, die an ihrem Molekülgerüst eine oder mehrere Sulfonsäuregruppen bzw. Sulfonatgruppen tragen, beruht.

Bornitrid (BN oder besser $B_{\infty}N_{\infty}$) ist ein homogenes Pulver, welches bekanntermaßen die Eigenschaften kosmetischer Pudergrundlagen verbessert. Im folgenden sind die Strukturen der beiden Hauptmodifikationen des Bornitrids schematisch angegeben, wobei auf die Angabe mesomerer Grenzstrukturen (beim hexagonalen $\alpha$-Bornitrid) sowie der formalen Ladungen (beim kubischen $\beta$-Bornitrid) verzichtet wurde.

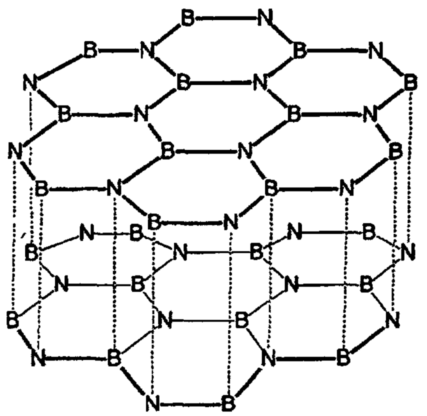

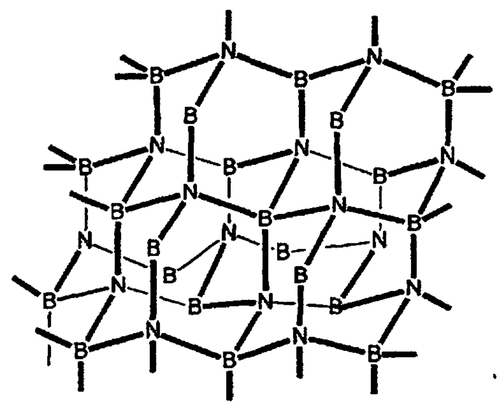

α-Bornitrid β-Bornitrid

Es war indes überraschend und für den Fachmann nicht vorauszusehen, daß die Verwendung von Bomitrid zur Erhöhung der Emulsionsstabilität kosmetischer oder dermatologischer Lichtschutzzubereitungen, welche sich durch einen Gehalt an wasserlöslichen UV-Filtersubstanzen, die an ihrem Molekülgerüst eine oder mehrere Sulfonsäuregruppen bzw. Sulfonatgruppen tragen, auszeichnen, den Nachteilen des Standes der Technik abhelfen würde.

Das α-Bornitrid stellt die erfindungsgemäß bevorzugt eingesetze Modifikation dar.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% Bornitrid

Die UV-A-Filtersubstanzen sind die Phenylen-1,4-bis-(2-benzimidazyl)-3,3'-5,5'-tetrasulfonsäure:

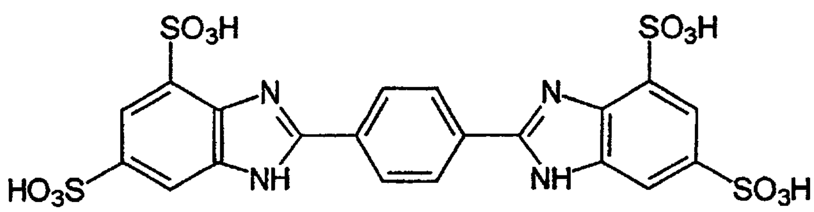

und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz (im Rahen dieser Offenbarung auch gelegentlich "Bisimidazylat" genannt):

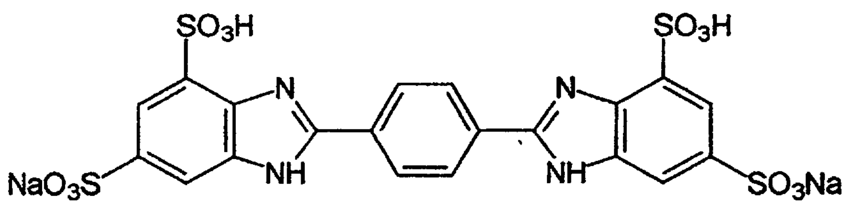

Weitere vorteilhaften UV-A-Filtersubstanzen sind das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bomylidenmethyl-10-sulfonsäure) (Im Rahen dieser Offenbarung auch gelegentlich "Bisimidazylat" genannt) bezeichnet wird und sich durch die foigende Struktur auszeichnet

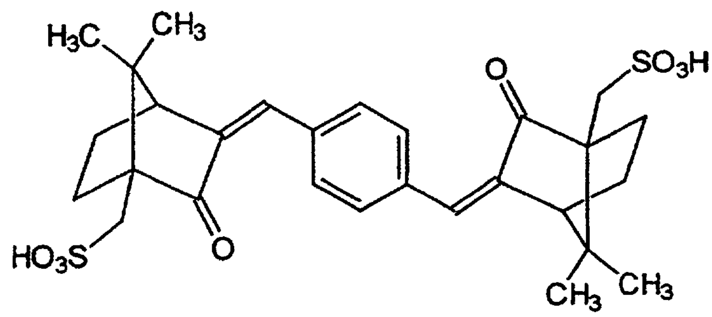

Gemäß der erfindungsgemäßen Verwendung einzusetzende Elektrolyte können vorteilhaft gewählt werden aus der Gruppe der Salze mit folgenden Anionen: Chloride, femer anorganische Oxo-Element-Anionen, von diesen insbesondere Sulfate, Carbonate, Phosphate, Borate und Aluminate. Auch auf organischen Anionen basierende Elektrolyte sind vorteilhaft, z.B. Lactate, Acetate, Benzoate, Propionate, Tartrate, Citrate, Aminosäuren und deren Salze und andere mehr. Vergleichbare Effekte sind auch durch Ethylendiamintetraessigsäure und deren Salze zu erzielen. Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat und Mischungen daraus.

Gemäß der erfindungsgemäßen Verwendung vorteilhaft einzusetzende Elektrolyte können ferner gewählt werden aus der Gruppe der kosmetisch und dermatologisch relevanten α-Hydroxycarbonsäuren, α-Ketocarbonsäuren und β-Hydroxycarbonsäuren und insbesondere deren Salze, wobei die Kationen vorteilhaft gewählt werden können aus der Gruppe Ammonium,- Alkylammonium,- Alkalimetall-, Erdalkalimetall,- Magnesium-, Eisen- bzw. Zinkionen.

α-Hydroxycarbonsäuren, welche kosmetisch oder dermatologisch relevant sind, folgen der allgemeinen Formel

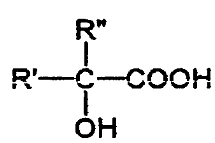

β-Hydroxycarbonsäuren, welche kosmetisch oder dermatologisch relevant sind, folgen der allgemeinen Formel

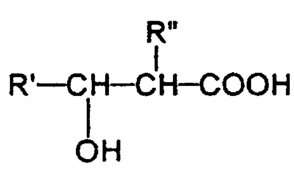

bzw.

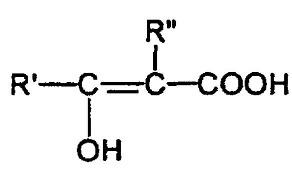

α-Ketocarbonsäuren, welche kosmetisch oder dermatologisch relevant sind, folgen der allgemeinen Formel

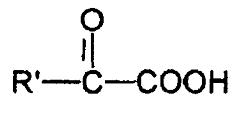

wobei jeweils R' und R" unabhängig voneinander gewählt werden aus der Gruppe

(a1) H- ,

(a2) verzweigtes oder unverzweigtes $C_{1-25}$-Alkyl-,
(a3) mit einer oder mehreren Carboxylgruppen und/oder Hydroxygruppen und/oder Aldehydgruppen und/oder Oxogruppen (Ketogruppen) substituiertes verzweigtes oder unverzweigtes $C_{1-25}$-Alkyl-
(a4) Phenyl-,
(a5) mit einer oder mehreren Carboxylgruppen und/oder Hydroxygruppen und/oder verzweigten und/oder unverzweigten $C_{1-25}$-Alkylgruppen substituiertes Phenyl-,
oder wobei das α-Kohlenstoffatom und das β-Kohlenstoffatom der β-Hydroxycarbonsäure mit R' und R" zusammen eine
(a6) unsubstituierte Cycloalkylgruppe oder Arylgruppe mit 3 bis 7 Ringatomen oder eine
(a7) mit einer oder mehreren Carboxylgruppen und/oder Hydroxygruppen und/oder Oxogruppen (Ketogruppen) und/oder verzweigten und/oder unverzweigten $C_{1-25}$-Alkylgruppen substituierte Cycloalkylgruppe oder Arylgruppe mit 3 bis 7 Ringatomen

ausbildet und
wobei die α-Hydroxycarbonsäuren oder die β-Hydroxycarbonsäuren oder die α-Ketocarbonsäuren gegebenenfalls in Form ihrer physiologisch verträglichen Salze vorliegen können.

Es folgen vorteilhaft im Rahmen der vorliegenden Erfindung zu verwendende α-Hydroxycarbonsäuren, β-Hydroxycarbonsäuren und α-Ketocarbonsäuren, wobei diese auch stellvertretend für ihre Salze bzw. Anionen aufgeführt werden:

Die Salicylsäure (auch 2-Hydroxybenzoësäure, Spirsäure), welche durch die Struktur

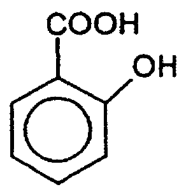

gekennzeichnet ist. Bekanntermaßen wirkt Salicylsäure antibakteriell und keratolytisch und ist Bestandteil mancher kosmetischen oder pharmazeutischen Zubereitungen.

Die den erfindungsgemäß verwendeten α-Hydroxycarbonsäuren werden vorteilhaft gewählt aus folgenden Substanzklassen:

(a2) α-Hydroxyfettsäuren, wobei diese wiederum besonders vorteilhaft aus der Gruppe der $C_{10-18}$-Alkylcarbonsäuren gewählt werden,
(a3) α-Hydroxyzuckersäuren, aliphatische α-Hydroxyfruchtsäuren,
(a4) unsubstituierte aromatische α-Hydroxycarbonsäuren (z.B. Mandelsäure) bzw.
(a5) substituierte aromatische α-Hydroxycarbonsäuren.

Die unter Punkt (a2) fallenden α-Hydroxyfettsäuren werden besonders vorteilhaft gewählt aus der Gruppe

- α-Hydroxycarbonsäuren, gemäß der Formel

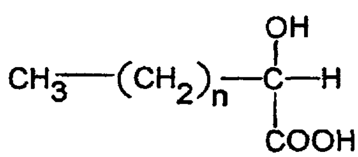

und/oder

- α-Hydroxy-isocarbonsäuren, gemäß der Formel

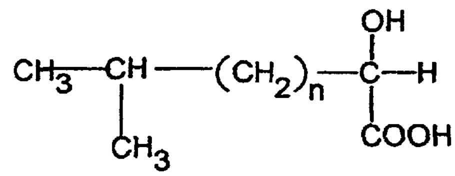

und/oder

- α-Hydroxy-anteisocarbonsäuren, gemäß der Formel

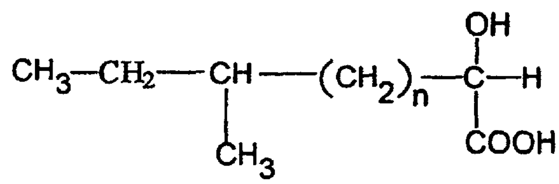

,

wobei n jeweils eine Zahl von 7 bis 31 darstellt.

Vorteilhaft ist weiter, Gemische solcher aliphatischen α-Hydroxycarbonsäuren, insbesondere in Form von Wollwachssäuregemischen zu verwenden, in welchen der Gehalt an α-Hydroxycarbonsäuren 20 - 30 Gew.-%, bezogen auf die Gesamtzusammensetzung beträgt.

Die unter Punkt (a3) fallenden α-Hydroxyzuckersäuren werden besonders vorteilhaft gewählt aus der Gruppe der

- Aldonsäuren, z.B. Gluconsäure, Galactonsäure
- Aldarsäuren, z.B. Glucarsäure, Galactarsäure (aber auch die Fruchtsäure Weinsäure, die ebenfalls unter die Definition der Aldarsäure fällt)
- Uronsäuren, z.B. Glucuronsäure, Galacturonsäure
- Glycerinsäure

Die unter Punkt (a3) fallenden aliphatischen α-Hydroxyfruchtsäuren werden besonders vorteilhaft gewählt aus der Gruppe Äpfelsäure, Milchsäure, Citronensäure, Weinsäure.

Äpfelsäure (Hydroxybemsteinsäure) ist durch folgende chemische Struktur gekennzeichnet:

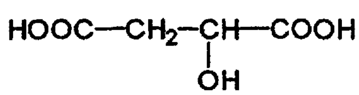

Milchsäure (2-Hydroxypropansäure) ist durch folgende chemische Struktur gekennzeichnet:

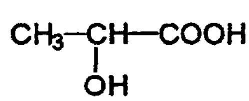

Citronensäure (2-Hydroxy-1,2,3-propantricarbonsäure) ist durch folgende chemische Struktur gekennzeichnet

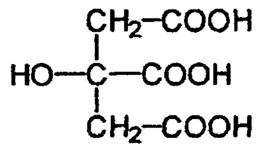

.

Bekanntermaßen wird Citronensäure zur Pufferung kosmetischer und/oder dermatologischer Zubereitungen, aber auch als Synergist für Antioxidantien in der Haut- und Haarkosmetik verwendet.

Weinsäure (Dihydroxybemsteinsäure) ist durch folgende chemische Struktur gekennzeichnet:

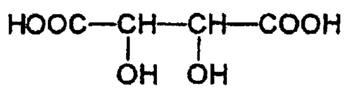

Bevorzugte α-Ketocarbonsäure ist die Brenztraubensäure (α-Oxopropansäure). Sie zeichnet sich durch folgende Struktur aus:

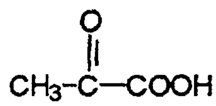

Die Höchstmenge der einzusetztenden Elektrolyte ist letztendlich abhängig von deren Löslichlichkeit in der wäßrigen Phase. Grundsetzlich setzt die erfindungsgemäße Lehre aber keine Höchstmengen als Schranke, da es gegebenenfalls ja sogar vorteilhaft sein mag, aus welchen Gründen auch immer, einen über die Löslichkeit eines Elektrolytes hinausgehende zusätzliche Menge dieses Elektrolytes, beispielsweise als ungelösten Festkörper, in eine kosmetische oder dermatologische Zubereitung einzuarbeiten.

Es ist besonders vorteilhaft, Bomitrid einzusetzen zum Erzielen oder Erhöhen der Stabilität von O/W-Emulsionen, gegenüber der Gegenwart von Elektrolyten, wobei die Emulsionen in mindestens einer der wäßrigen Phasen einen oder mehrere Elektrolyte gelöst enthält, und die Ionenstärke der wäßrigen Phasen, in der der oder die Elektrolyte gelöst vorliegen, mindestens 0,0025, vorzugsweise mindestens 0,005, insbesondere mindestens 0,0075 mol/l beträgt.

Es kann auch vorteilhaft sein, Bornitrid einzusetzen zum Erzielen oder Erhöhen der Stabilität von O/W-Emulsionen, gegenüber der Gegenwart von Elektrolyten, wobei die Emulsionen 0,1 bis 20 Gew.-%, vorteilhaft 0,5 bis 10 Gew.-%, ganz besonders bevorzugt 1 bis 5 Gew.-% an Elektrolyten enthalten.

Die Gesamtmenge an wasserlöslichen UV-Filtersubstanzen in den fertigen kosmetischen oder dermatologischen Zubereitungen, beispielsweise an 2-Phenylbenzimidazol-5-sulfonsäure bzw. deren Salzen und/oder 2-Hydroxy-4-methoxybenzophenon-5-sulfonsäure bzw. deren Salzen und/oder 4-(2-Oxo-3-bornylidenmethyl)benzolsulfonsäure bzw. deren Salzen und/oder 2-Methyl-5-(2-oxo-3-bornylidenmethyl)benzolsulfonsäure bzw. deren Salzen und/oder Benzol-1,4-di(2-oxo-3-bomylidenmethyl)-10-sulfonsäure bzw. deren Salzen, wird vorteilhaft aus dem Bereich von 0,1 - 10,0 Gew.-%, bevorzugt 0,5 - 6,0 Gew.-% gewählt, bezogen auf das Gesamtgewicht der Zubereitungen, falls die Gegenwart dieser Substanzen erwünscht ist.

Weitere vorteilhafte UV-A-Filtersubstanzen im Sinne der vorliegenden Erfindung sind Dibenzoylmethanderivate, insbesondere 5-Isopropyldibenzoylmethan (CAS-Nr. 63250-25-9), welches sich durch die Struktur

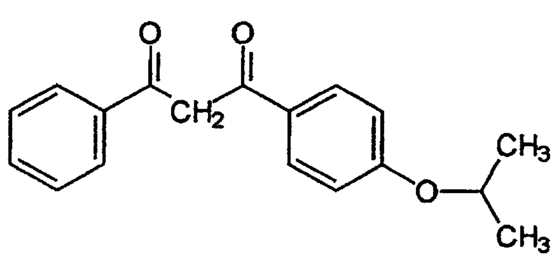

auszeichnet und von Merck unter der Marke Eusolex® 8020 verkauft wird, und/oder das 4-(tert.-Butyl)-4'-methoxydibenzoylmethan (CAS-Nr. 70356-09-1), welches sich durch die Struktur

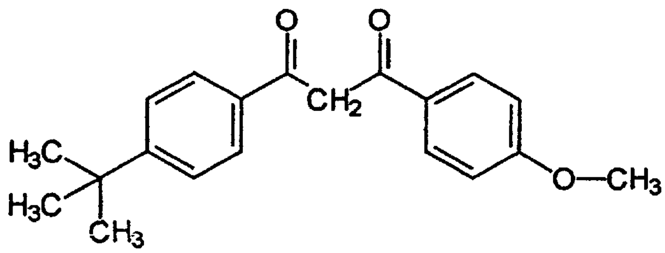

auszeichnet und von Givaudan unter der Marke Parsol® 1789 verkauft wird.

Weitere im Sinne der vorliegenden Erfindung vorteilhaft einzusetzende Lichtschutzfiltersubstanzen weisen das Strukturelement des Methylidencamphers auf, so beispielsweise der 4-Methylbenzylidencampher, welcher sich durch die Struktur

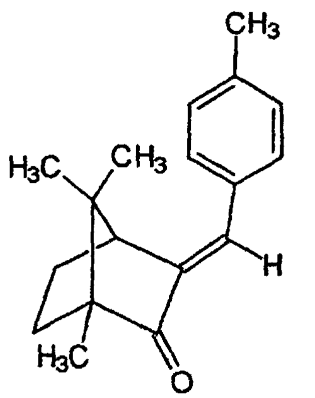

auszeichnet und von Merck unter der Marke Eusolex® 6300 verkauft wird, sowie der Benzylidencampher, welcher sich durch die Struktur

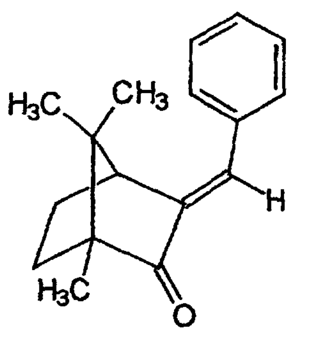

auszeichnet und von der Gesellschaft Induchem unter der Marke Unisol® S22 verkauft wird.

Weitere im Sinne der vorliegenden Erfindung vorteilhaft einzusetzende Lichtschutzfiltersubstanzen weisen das Strukturelement des Benzophenons auf:

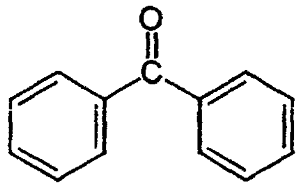

Vorteilhaft können erfindungsgemäß die Substanzen, die das Strukturmotiv des Benzophenons enthalten (in dieser Schrift auch als "Benzophenone" bezeichnet) gewählt werden aus der Gruppe der folgenden Substanzen

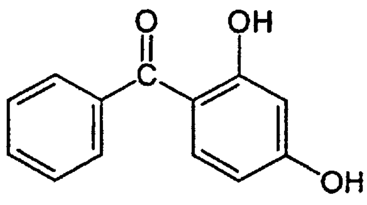

Benzophenon-1
(2,4-Dihydroxybenzophenon)
CAS-Nr. 131-56-6

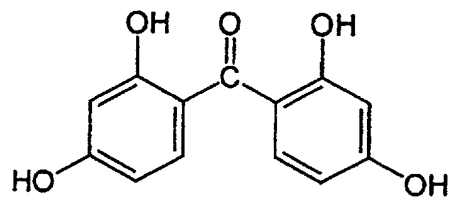

Benzophenon-2
(2,2',4,4'-Tetrahydroxybenzophenon)
CAS-Nr. 131-55-5

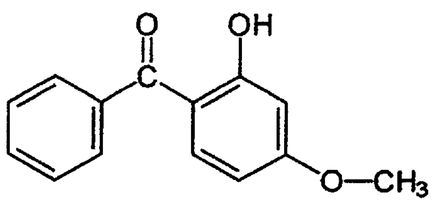

Benzophenon-3
(2-Hydroxy-4-methoxybenzophenon)

CAS-Nr. 131-57-7

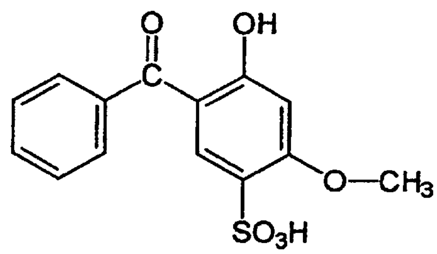

Benzophenon-4
(2-Hydroxy-4-methoxybenzophenon-5-sulfonsäure)
CAS-Nr. 4065-45-6

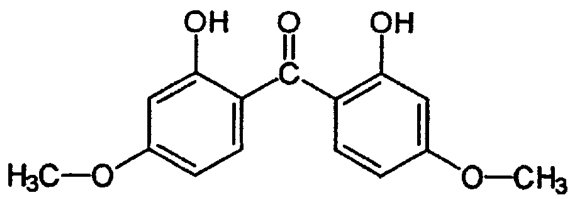

Benzophenon-6
(2,2'-Dihydroxy-4,4'-dimethoxybenzophenon)

CAS-Nr. 131-54-4

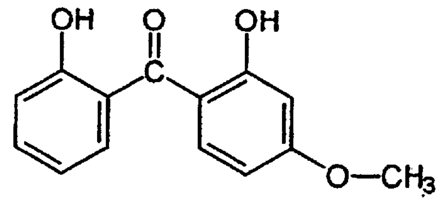

Benzophenon-8
(2,2'-Dihydroxy-4-methoxybenzophenon)
CAS-Nr. 131-53-3

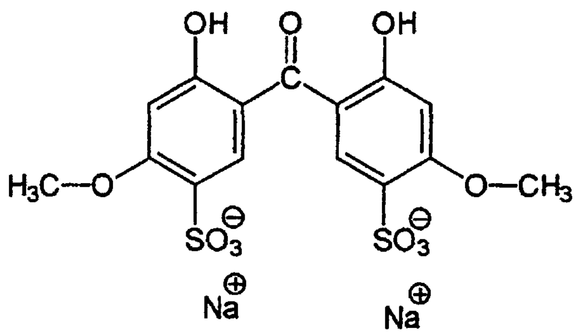

Benzophenon-9
(Dinatrium-2,2'-dihydroxy-4,4'-dimethoxy-5,5'-disulfobenzophenon)
Cas-Nr. 3121-60-6,

ferner Benzophenon-11, CAS-Nr. 1341-54-4.

Bevorzugtes Benzophenon ist Benzophenon-3, welches beispielsweise von Merck unter der Warenbezeichnung Eusolex® 4360 verkauft wird.

Von den Dibenzoylmethanderivaten werden vorteilhaft verwendet:

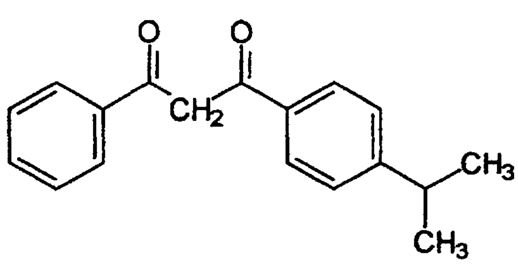

4-Isopropyldibenzoylmethan
CAS-Nr. 63260-25-9

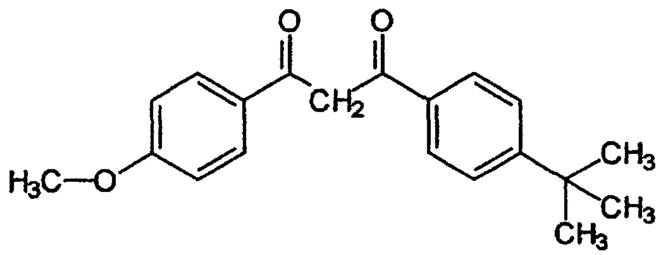

**4-(tert.-Butyl)-4'-methoxydibenzoylmethan**

**CAS-Nr. 70356-09-1**

Weitere im Sinne der vorliegenden Erfindung vorteilhaft einzusetzende Lichtschutzfiltersubstanzen sind Salicylsäurederivate wie

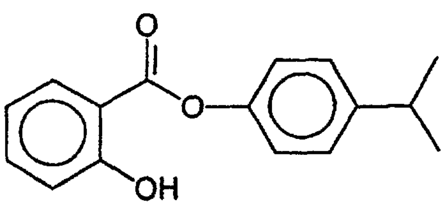

**(4-Isopropylbenzylsalicylat),**

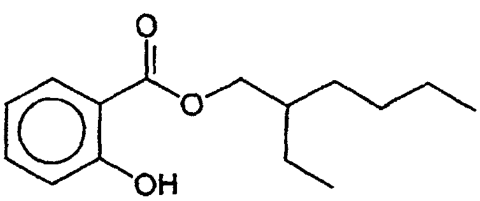

**(2-Ethylhexylsalicylat, Octylsalicylat),**

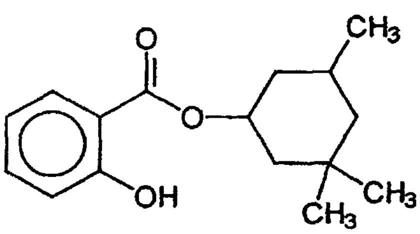

**(Homomenthylsalicylat).**

Weiterere im Sinne der vorliegenden Erfindung vorteilhafte Lichtschutzfiltersubstanz sind Zimtsäureester, beispielsweise das 2-Ethylhexyl-p-methoxy-cinnamat (4-Methoxyzimtsäure-2'ethylhexylhester), welches von Givaudan unter der Bezeichnung Parsol® MCX erhältlich ist und sich durch folgende Struktur auszeichnet

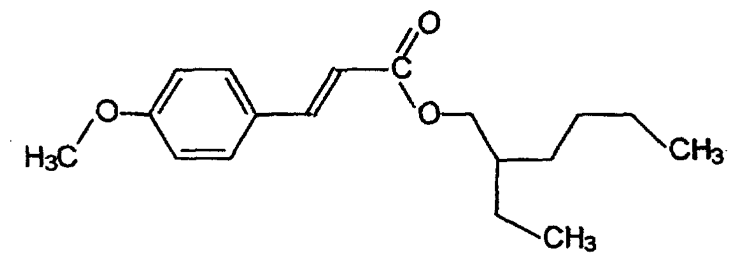

Vorteilhafte UV-Filtersubstanzen im Sinne der vorliegenden Erfindung sind femer sogenannte Breitbandfilter, d.h. Filtersubstanzen, die sowohl UV-A- als auch UV-B-Strahlung absorbieren.

Vorteilhafte Breitbandfilter sind beispielsweise Bis-Resorcinyltriazinderivate mit der folgenden Struktur

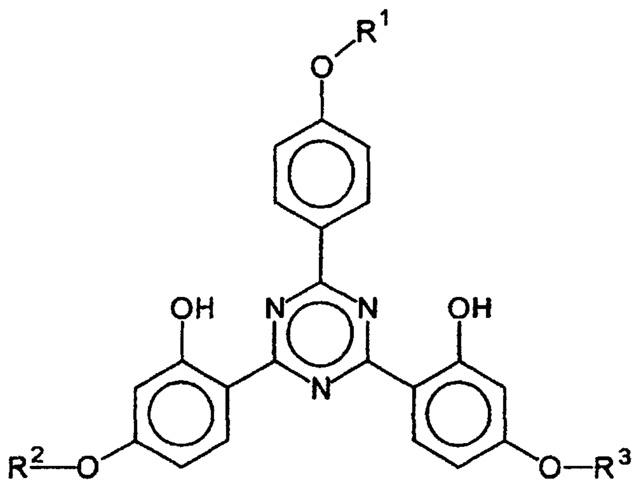

wobei $R^1$, $R^2$ und $R^3$ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt ist das 2,4-Bis-{[4-(2-Ethylhexyloxy)-2-hydroxy]-phenyl}-6-(4-methoxyphenyl)-1,3,5-triazin, welches durch folgende Struktur gekennzeichnet ist

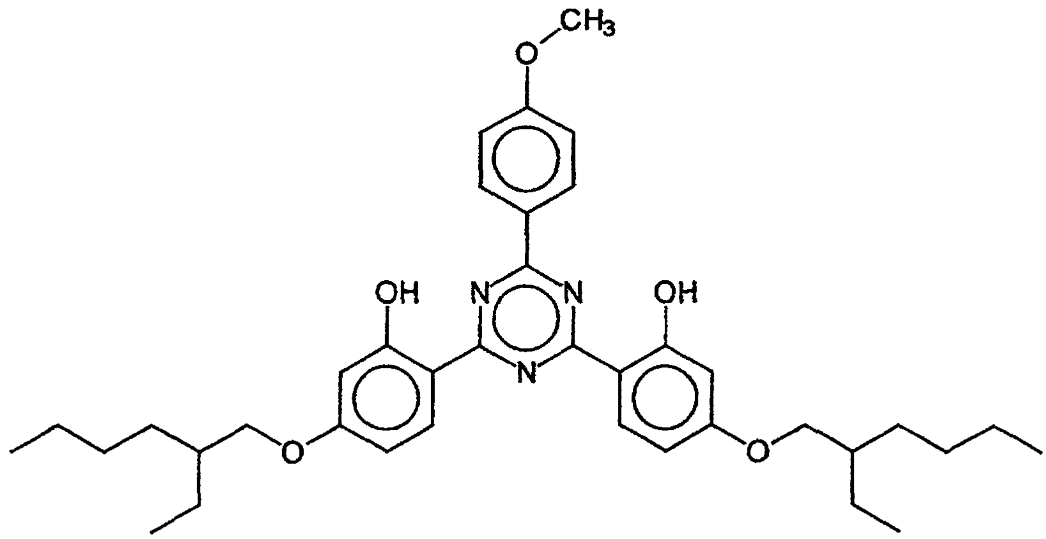

Ein weiterer vorteilhafter UV-Filter ist der 4,4',4"-(1,3,5-Triazin-2,4,6-triyltriimino)-tris-benzoësäure-tris (2-ethylhexylester), synonym: 2,4,6-Tris-[anilino-(p-carbo-2'-ethyl-1'-hexyloxy)]-1,3,5-triazin.

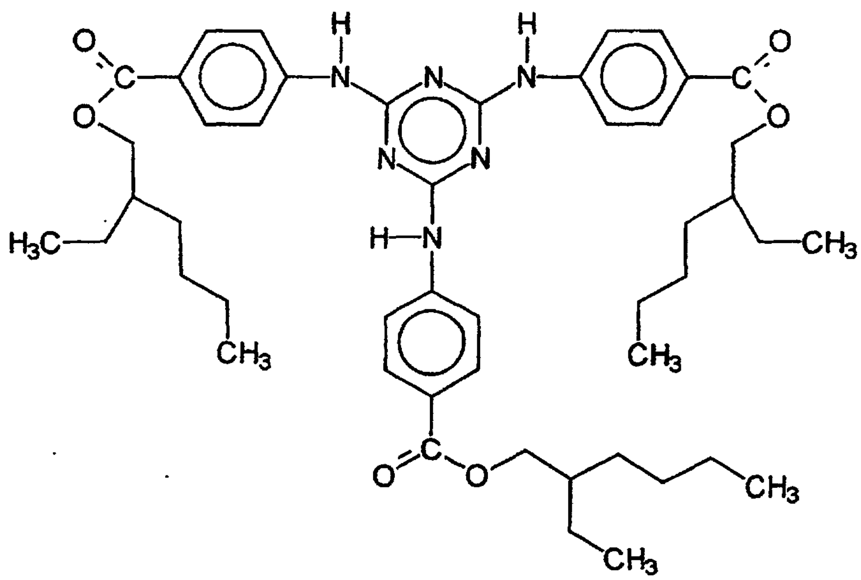

Diese UV-B-Filtersubstanz wird von der BASF Aktiengesellschaft unter der Warenbezeichnung UVINUL® T 150 vertrieben und zeichnet sich durch gute UV-Absorptionseigenschaften aus. Der Hauptnachteil des 4,4', 4"-(1,3,5-Triazin-2,4,6-triyltriimino)-tris-benzoësäure-tris(2-ethylhexylesters) ist seine schlechte Löslichkeit in Lipiden. Es bereitete daher in der Vergangenheit gewisse formulierungstechnische Schwierigkeiten, mit Hilfe dieses Filters höhere Lichtschutzfaktoren zu erzielen, was aber durch die Lehre der Erfindung überkommen werden konnte.

Zwischenzeitlich wurden von verschiedenen Autoren auch andere UV-Filtersubstanzen beschrieben, welche das Strukturmotiv

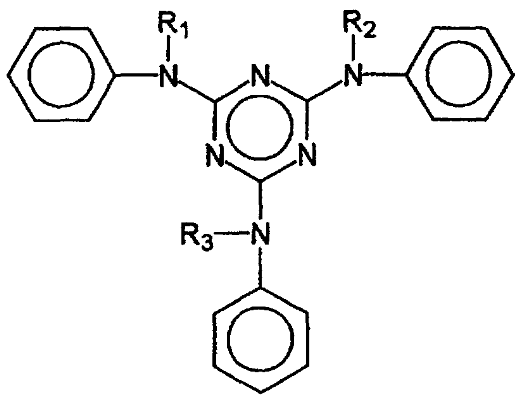

aufweisen und deren Einarbeitung in kosmetische oder dermatologische Lichtschutzformulierungen zumindest gewisse Probleme aufweisen kann. So werden in der Europäischen Offenlegungsschrift 570838 s-Triazinderivate beschrieben, deren chemische Struktur durch die generische Formel

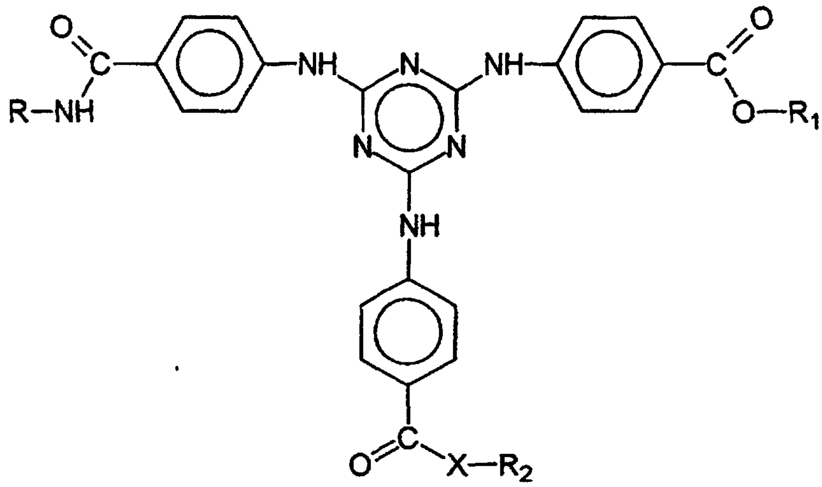

wiedergegeben wird, wobei

R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,

X ein Sauerstoffatom oder eine NH-Gruppe darstellt,

$R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

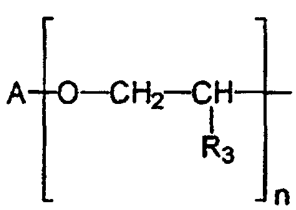

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
$R_3$ ein Wasserstoffatom oder eine Methylgruppe darstellt,
n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

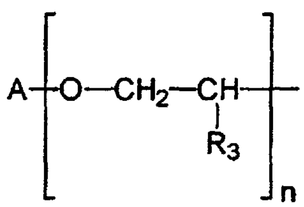

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
$R_3$ ein Wasserstoffatom oder eine Methylgruppe darstellt,
n eine Zahl von 1 bis 10 darstellt,
wenn X ein Sauerstoffatom darstellt.

Ein Beispiel für solche unsymmetrisch substituierte s-Triazine stellt das Dioctylbutylamidotriazon dar, dessen chemische Struktur durch die Formel

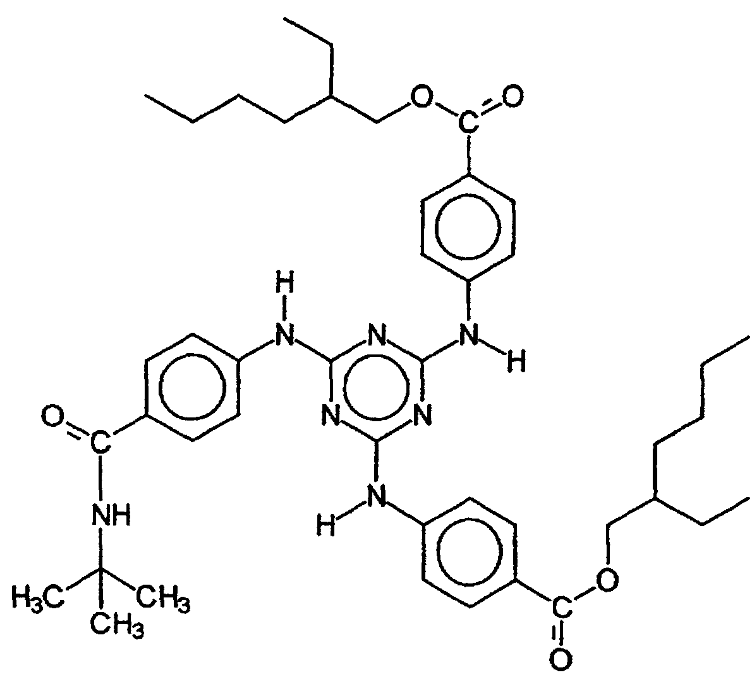

wiedergegeben wird.

Auch andere UV-Filtersubstanzen, deren Einarbeitung in kosmetische oder dermatologische Lichtschutzformulierungen Probleme aufweist, sind bekannt. So werden in der Europäischen Offenlegungsschrift 775698 Bis-Resorcinyltriazinderivate beschrieben, deren chemische Struktur durch die generische Formel

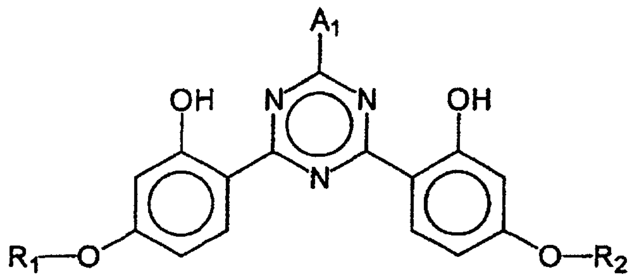

wiedergegeben wird, wobei $R_1$, $R_2$ und $A_1$ verschiedenste organische Reste repräsentieren.

Ferner vorteilhaft im Sinne der vorliegenden Erfindung ist das 2,4-Bis-{[4-(3-sulfonato)-2-hydroxy-propyloxy)-2-hydroxy]-phenyl}-6-(4-methoxyphenyl)-1,3,5-triazin Natriumsalz, welches durch folgende Struktur gekennzeichnet ist:

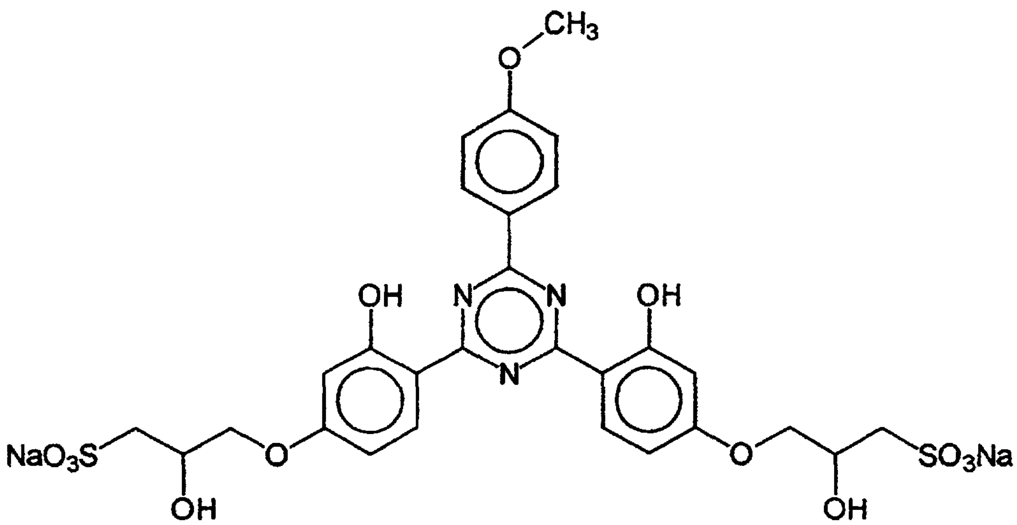

Ferner vorteilhaft ist das 2,4-Bis-{[4-(3-(2-Propyloxy)-2-hydroxy-propyloxy)-2-hydroxy]-phenyl}-6-(4-methoxyphenyl)-1,3,5-triazin, welches durch folgende Struktur gekennzeichnet ist:

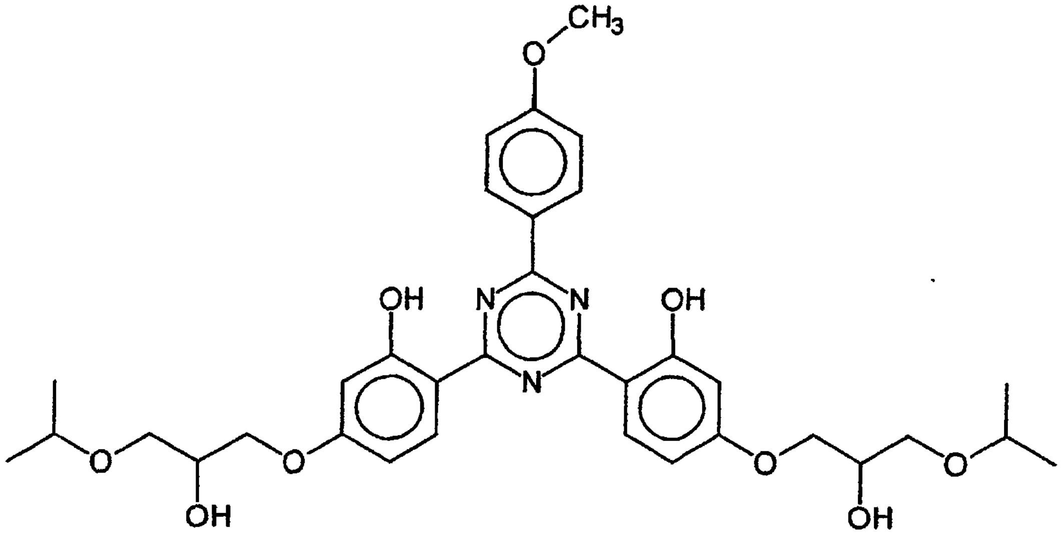

Ferner vorteilhaft ist das 2,4-Bis-{[4-(2-ethyl-hexyloxy)-2-hydroxy]-phenyl}-6-[4-(2-methoxyethyl-carboxyl)-phenylamino]-1,3,5-triazin, welches durch folgende Struktur gekennzeichnet ist:

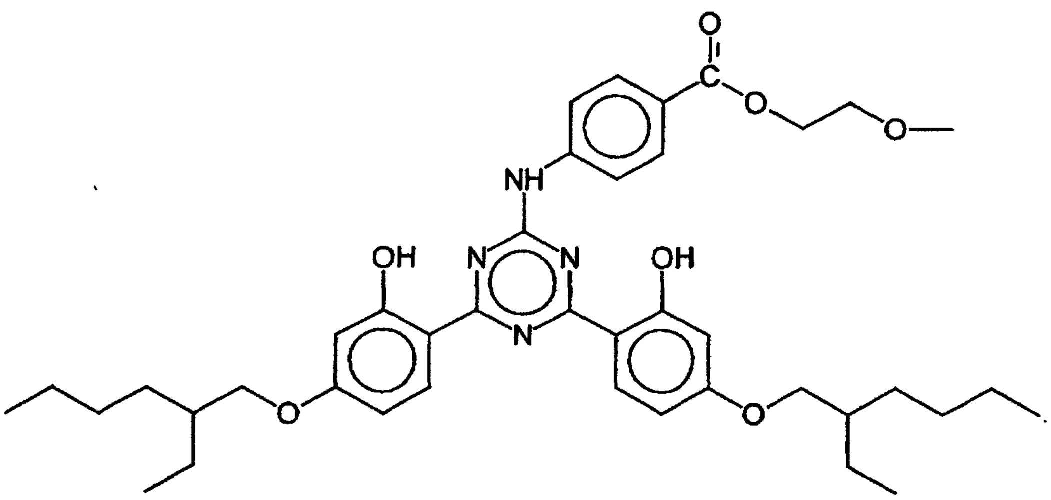

Ferner vorteilhaft ist das 2,4-Bis-{[4-(3-(2-propyloxy)-2-hydroxy-propy(oxy)-2-hydroxy]-phenyl}-6-[4-(2-ethyl-carboxyl)-phenylamino]-1,3,5-triazin, welches durch folgende Struktur gekennzeichnet ist:

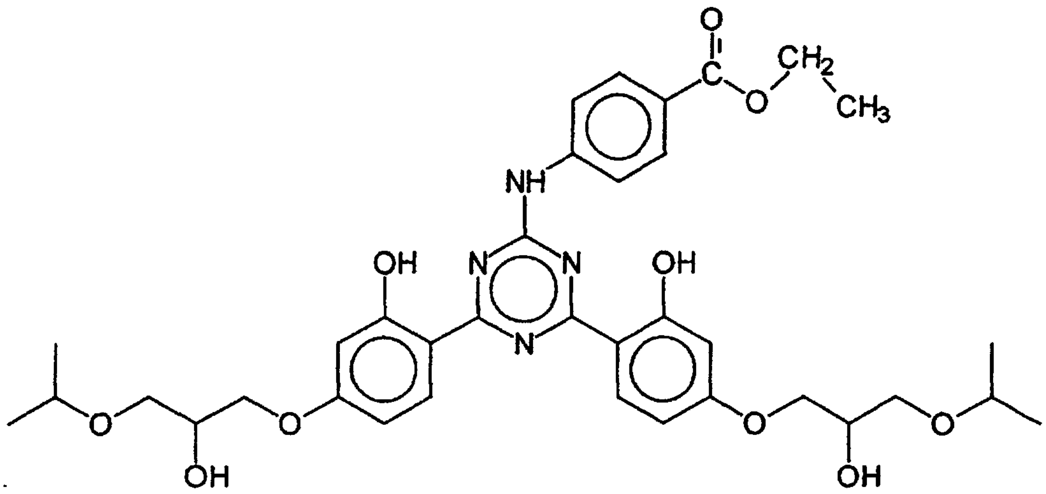

Ferner vorteilhaft ist das 2,4-Bis-{[4-(2-ethyl-hexyloxy)-2-hydroxy]-phenyl}-6-(1-methylpyrrol-2-yl)-1,3,5-triazin, welches durch folgende Struktur gekennzeichnet ist

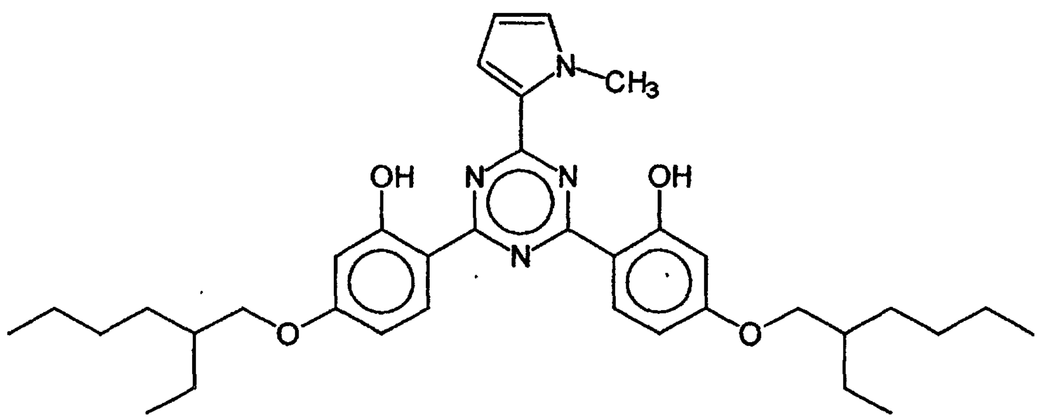

Ferner vorteilhaft ist das 2,4-Bis-{[4-tris(trimethylsiloxy-silylpropyloxy)-2-hydroxy]-phenyl}-6-(4-methoxyphenyl)-1,3,5-triazin, welches durch folgende Struktur gekennzeichnet ist

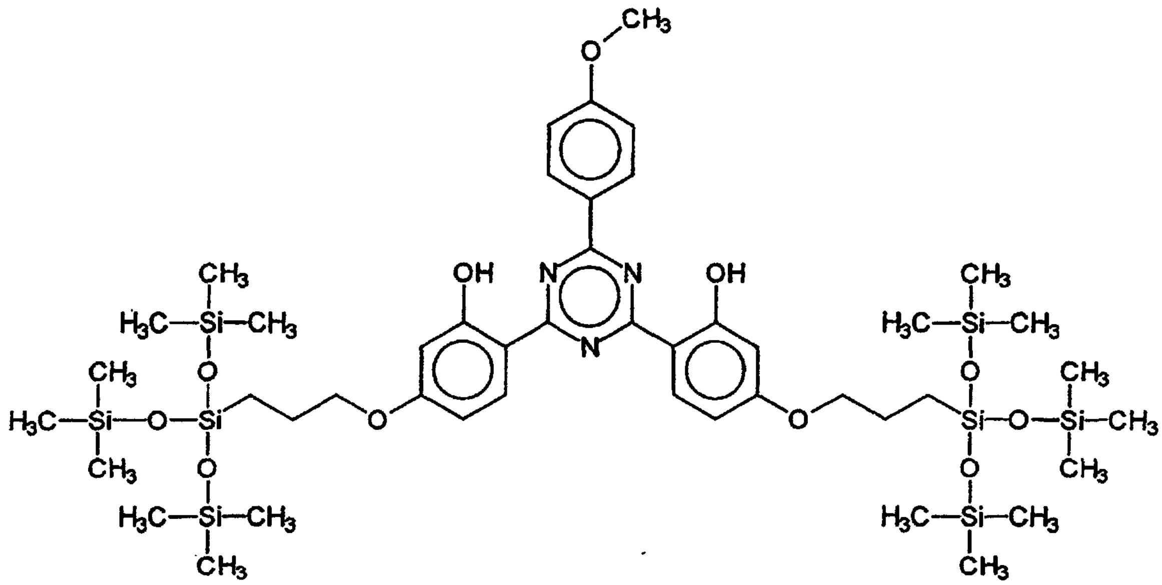

Ferner vorteilhaft ist das 2,4-Bis-{[4-(2"-methylpropenyloxy)-2-hydroxy]-phenyl}-6-(4-methoxyphenyl)-1,3,5-triazin, welches durch folgende Struktur gekennzeichnet ist:

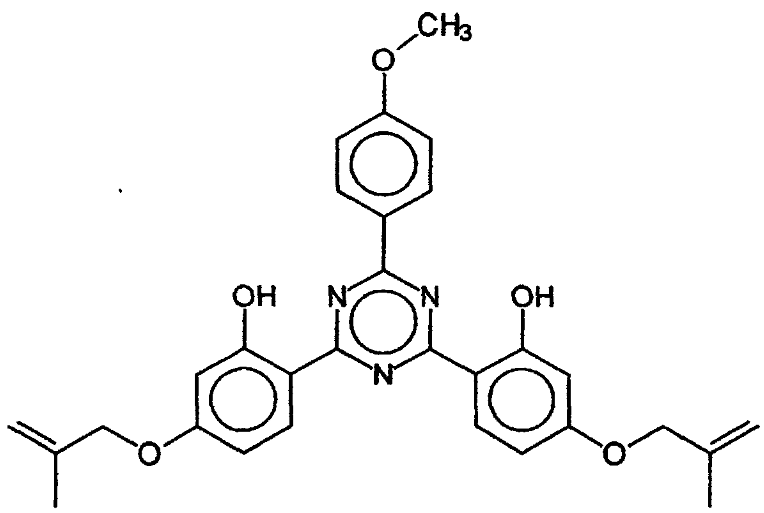

Ferner vorteilhaft ist das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin, welches durch folgende Struktur gekennzeichnet ist:

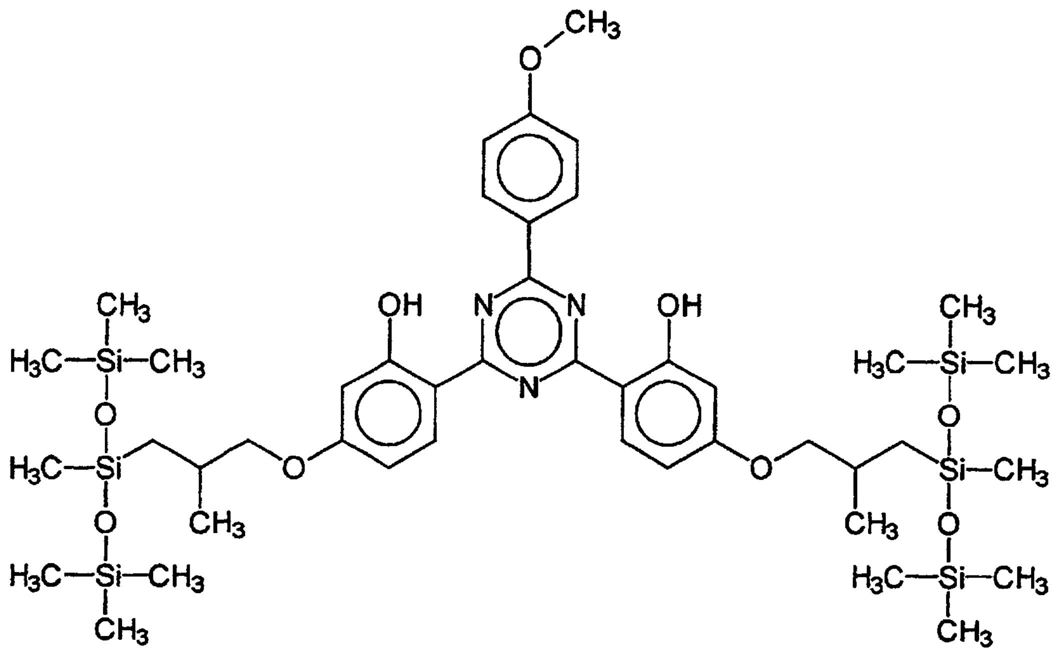

Noch ein weiterer vorteilhafter UV-Filter ist das 2,2'-Methylen-bis-(6-(2H-benzotriazol-2-yl)-4-(1,1,3,3-tetramethylbutyl)-phenol), welches durch die chemische Strukturformel

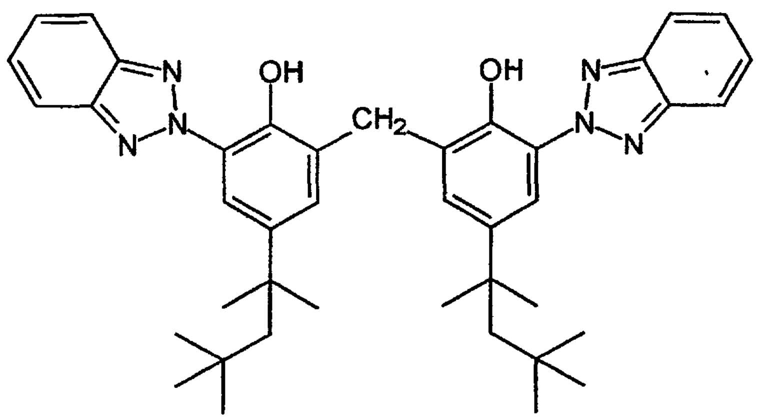

gekennzeichnet ist.

Die gemäß der Erfindung einsetzbaren UV-B-Filter können öllöslich oder wasserlöslich. sein. Vorteilhafte öllösliche UV-B-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;

Im Sinne der Erfindung vorteilhafte wasserlösliche UV-B-Filtersubstanzen sind z.B.:

- Salze der 2-Phenylbenzimidazol-5-sulfonsäure, wie ihr Natrium-, Kalium- oder ihr Triethanolammonium-Salz, sowie die Sulfonsäure selbst;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und deren Salze.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Insbesondere war erstaunlich, daß Bornitrid im Sinne der vorliegenden Erfindung zu stabilen kosmetischen oder dermatologischen Emulsionen führen würde, wenn wenigstens einer der UV-Filter in den Lichtschutzzubereitungen gemäß der vorliegenden Erfindung aus der Gruppe der Triazinderivate gewählt wird, insbesondere gewählt aus der Gruppe

- 4,4',4''-(1,3,5-Triazin-2,4,6-triyltriimino)-tris-benzoesäure-tris(2-ethylhexylester)
- 2,4-Bis-{[4-(2-Ethyl-hexyloxy)-2-hydroxy]-phenyl}-6-(4-methoxyphenyl)-1,3,5-triazin,
- 2,4-Bis-{[4-(3-sulfonato)-2-hydroxy-propyloxy)-2-hydroxy]-phenyl}-6-(4-methoxyphenyl)-1,3,5-triazin Natriumsalz,
- 2,4-Bis-{[4-(3-(2-Propyloxy)-2-hydroxy-propyloxy)-2-hydroxy]-phenyl}-6-(4-methoxyphenyl)-1,3,5-triazin,
- 2,4-Bis-{[4-(2-ethyl-hexyloxy)-2-hydroxy]-phenyl}-6-[4-(2-methoxyethyl-carboxyl)-phenylamino]-1,3,5-triazin,
- 2,4-Bis-{[4-(3-(2-propyloxy)-2-hydroxy-propyloxy)-2-hydroxy]-phenyl}-6-[4-(2-ethylcarboxyl)-phenylamino]-1,3,5-triazin,
- 2,4-Bis-{[4-(2-ethyl-hexyloxy)-2-hydroxy]-phenyl}-6-(1-methyl-pyrrol-2-yl)-1,3,5-triazin,
- 2,4-Bis-{[4-tris(trimethylsiloxy-silylpropyloxy)-2-hydroxy]-phenyl}-6-(4-methoxyphenyl)-1,3,5-triazin,
- 2,4-Bis-{[4-(2''-methylpropenyloxy)-2-hydroxy]-phenyl}-6-(4-methoxyphenyl)-1,3,5-triazin,
- 2,4-Bis-{[4-(1',1',1',3',5',5',5'-Heptamethylsiloxy-2''-methyl-propyloxy)-2-hydroxy]-phenyl}-6-(4-methoxyphenyl)-1,3,5-triazin.

Die Liste der genannten UV-Filter, die im Sinne der vorliegenden Erfindung eingesetzt werden können, soll selbstverständlich nicht limitierend sein.

Die Gesamtmenge an öllöslichen UV-Filtersubstanzen in den fertigen kosmetischen oder dermatologischen Zubereitungen, beispielsweise an 4,4',4''-(1,3,5-Triazin-2,4,6-triyltriimino)-tris-benzoesäure-tris(2-ethylhexylester) und/oder 4-(tert.-Butyl)-4'-methoxydibenzoylmethan und/oder 4-Methylbenzylidencampher, wird vorteilhaft aus dem Bereich von 0,1 - 20,0 Gew.-%, bevorzugt 0,1 - 10,0 Gew.-% gewählt, bezogen auf das Gesamtgewicht der Zubereitungen, falls die Gegenwart dieser Substanzen erwünscht ist

Die Gesamtmenge an 2-Ethylhexyl-p-methoxy-cinnamat in den fertigen kosmetischen oder dermatologischen Zubereitungen wird, falls die Gegenwart dieser Substanz erwünscht ist, vorteilhaft aus dem Bereich von 0,1 - 15,0 Gew.-%, bevorzugt 0,5 - 7,5 Gew.-% gewählt, bezogen auf das Gesamtgewicht der Zubereitungen.

Die Gesamtmenge an Ethylhexyl-2-cyano-3,3-diphenylacrylat in den fertigen kosmetischen oder dermatologischen Zubereitungen wird, falls die Gegenwart dieser Substanz erwünscht ist, vorteilhaft aus dem Bereich von 0,1 - 15,0 Gew.-%, bevorzugt 0,5 - 10,0 Gew.-% gewählt, bezogen auf das Gesamtgewicht der Zubereitungen.

Die Gesamtmenge an einem oder mehreren Salicylsäurederivaten in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 2,5 Gew.-% zu wählen. Wenn Homomenthylsalicylat gewählt wird, ist es von Vorteil, dessen Gesamtmenge aus dem Bereich von 0,1 - 10,0 Gew.-%, bevorzugt 0,5 - 5,0 Gew.-% zu wählen.

Kosmetische und dermatologische Formulierungen im Sinne der Erfindung enthalten einen oder mehrere übliche UV-A-, UV-B- und/oder Breitbandfilter als Einzelsubstanzen oder in beliebigen Gemischen untereinander, in der Lipidphase und/oder in der wäßrigen Phase.

Die Gesamtmenge an UV-Filtersubstanzen (UV-A-, UV-B- und/oder Breitbandfilter) in den fertigen kosmetischen oder dermatologischen Zubereitungen, sei es als Einzelsubstanz oder in beliebigen Gemischen untereinander, wird vorteilhaft aus dem Bereich von 0,1 bis 30 Gew.-%, bevorzugt 0,1 bis 20,0 Gew.-%, insbesondere 0,5 bis 10,0 Gew.-% gewählt, bezogen auf das Gesamtgewicht der Zubereitungen.

Erfindungsgemäße kosmetische und dermatologische Zubereitungen enthalten femer vorteilhaft, wenngleich nicht zwingend, anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Diese Pigmente sind röntgenamorph oder nicht-röntgenamorph. Besonders bevorzugt handelt es sich um Pigmente auf der Basis von $TiO_2$.

Röntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente durch Flammenreaktion erhältlich, beispielsweise dadurch, daß ein Metall- oder Halbmetallhalogenid mit Wasserstoff und Luft (oder reinem Sauerstoff) in einer Flamme umgesetzt wird.

In kosmetischen, dermatologischen oder pharmazeutischen Formulierungen werden röntgenamorphe Oxidpigmente als Verdickungs- und Thixotropierungsmittel, Fließhilfsmittel, zur Emulsions- und Dispersionsstabilisierung und als Trägersubstanz (beispielsweise zur Volumenerhöhung von feinteiligen Pulvern oder Pudem) eingesetzt.

Bekannte und in der kosmetischen oder dermatologischen Galenik oftmals verwendete röntgenamorphe Oxidpigmente sind die Siliciumoxide des Typs Aerosil® (CAS-Nr. 7631-86-9. Aerosile®, erhältlich von der Gesellschaft DEGUSSA, zeichnen sich durch geringe Partikelgröße (z.B. zwischen 5 und 40 nm) aus, wobei die Partikel als kugelförmige Teilchen sehr einheitlicher Abmessung anzusehen sind. Makroskopisch sind Aerosile® als lockere, weiße Pulver erkenntlich. Im Sinne der vorliegenden Erfindung sind röntgenamorphe Siliciumdioxidpigmente besonders vorteilhaft, und unter diesen gerade solche des Aerosil®-Typs bevorzugt.

Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974, Aerosil® R976.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% röntgenamorphe Oxidpigmente.

Die nicht-röntgenamorphen anorganischen Pigmente liegen erfindungsgemäß vorteilhaft in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter der Handelsbezeichnung T 805, vorteilhafte $TiO_2$/$Fe_2O_3$-Mischoxide unter der Handelsbezeichnung T 817 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und/oder dermatologischen Formulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen. Entsprechend können die erfindungsgemäßen Zubereitungen, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zubereitungen als Grundlage für pharmazeutische Formulierungen zu verwenden. Bevorzugt sind insbesondere solche kosmetischen und dermatologischen Zubereitungen, die in der Form eines Hautpflege- bzw. Schminkproduktes vorliegen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich zu erfindungsgemäß verwendeten UV-A-, UV-B- und/oder Breitbandfiltern mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Die jeweils einzusetzenden Mengen an kosmetischen oder dermatologischen Hilfs- und Trägerstoffen und

Parfüm können in Abhängigkeit von der Art des jeweiligen Produktes vom Fachmann durch einfaches Ausprobieren leicht ermittelt werden.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Camosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), femer (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-Eacetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 0,1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter

und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkemöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind femer Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, femer Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispiel 1**

| O/W - Emulsion | Gew.-% |
|---|---|
| Stearinsäure | 1,50 |
| Glycerinmonostearat | 3,00 |
| Caprylsäure/Caprinsäuretriglycerid | 10,00 |
| Dicaprylylether | 5,00 |
| Dimethicon | 2,00 |
| Hydriertes Polyisobuten | 2,00 |
| Vitamin-E-Acetat | 0,50 |
| Bornitrid | 4,00 |
| Octyltriazon | 1,00 |
| Methylbenzylidencampher | 4,00 |
| Butylmethoxydibenzoylmethan | 2,00 |
| Titandioxid | 1,00 |

(fortgesetzt)

| O/W - Emulsion | Gew.-% |
|---|---|
| Konservierung | 0,50 |
| Glycerin | 3,00 |
| Xanthan Gummi | 0,30 |
| Bisimidazylat | 1,00 |
| Natronlauge 45% | 0,50 |
| Wasser | ad 100,00 |

**Beispiel 2**

| O/W - Emulsion | Gew.-% |
|---|---|
| Stearinsäure | 1,50 |
| Glycerinmonostearat | 3,00 |
| Caprylsäure/Caprinsäuretriglycerid | 5,00 |
| Dicaprylylether | 5,00 |
| Dimethicon | 1,00 |
| Isohexadekan | 2,00 |
| Butylenglycoldicaprylat/caproat | 2,00 |
| $C_{12-15}$-Alkylbenzoate | 3,00 |
| Vitamin-E-Acetat | 0,50 |
| Bornitrid | 2,00 |
| Methylbenzylidencampher | 4,00 |
| Konservierung | 0,50 |
| Glycerin | 5,00 |
| Pemulen® TR1 | 0,20 |
| Phenylbenzimidazolsulfonsäure | 3,00 |
| Natronlauge 45% | 1,50 |
| Wasser | ad 100,00 |

**Beispiel 3**

| O/W - Emulsion | Gew.-% |
|---|---|
| Sorbitanstearat | 3,00 |
| Polyglyceryl-3-methylglucosedistearat | 1,50 |
| Octyldodecanol | 10,00 |
| Dicaprylylether | 5,00 |
| Cetylstearylisononanoat | 2,00 |
| Butylenglycoldicaprylat/caproat | 5,00 |
| Vitamin-E-Acetat | 0,50 |
| Bornitrid | 1,50 |
| Octyltriazon | 4,00 |
| Methylbenzylidencampher | 4,00 |
| Butylmethoxydibenzoyl methan | 3,00 |
| Konservierung | 0,50 |
| Glycerin | 10,00 |
| Xanthan Gummi | 0,20 |

(fortgesetzt)

| O/W - Emulsion | Gew.-% |
|---|---|
| Pemulen® TR1 | 0,10 |
| Bisimidazylat | 2,00 |
| Natronlauge 45% | 0,80 |
| Wasser | ad 100,00 |

**Beispiel 4**

| O/W - Emulsion | Gew.-% |
|---|---|
| Sorbitanstearat | 3,00 |
| Polyglyceryl-3-methylglucosedistearat | 1,50 |
| Octyldodecanol | 10,00 |
| Dimethicon | 2,00 |
| Mineralöl | 5,00 |
| Hydriertes Polyisobuten | 5,00 |
| Butylenglycoldicaprylat/caproat | 5,00 |
| Vitamin-E-Acetat | 0,50 |
| Bornitrid | 1,50 |
| Octyltriazon | 4,00 |
| Titandioxid | 5,00 |
| Konservierung | 0,50 |
| Glycerin | 10,00 |
| Xanthan Gummi | 0,20 |
| Pemulen® TR1 | 0,10 |
| Phenylbenzimidazolsulfonsäure | 1,00 |
| Bisimidazylat | 1,00 |
| Natronlauge 45% | 0,80 |
| Wasser | ad 100,00 |

**Beispiel 5**

| W/O-Emulsion | Gew.-% |
|---|---|
| Polyglyceryl-2-dipolyhydroxystearat | 5,00 |
| Dimethicon | 2,00 |
| Mineralöl | 5,00 |
| Isohexadekan | 5,00 |
| Butylenglycoldicaprylat/caproat | 5,00 |
| Dioctylbutamidotriazon | 3,00 |
| Bornitrid | 3,00 |
| Methylbenzylidencampher | 2,00 |
| Butylmethoxydibenzoyl methan | 2,00 |
| Titandioxid | 4,00 |
| Konservierung | 0,50 |
| Glycerin | 5,00 |
| $MgSO_4$ | 1,00 |
| Bisimidazylat | 1,00 |

(fortgesetzt)

| W/O-Emulsion | Gew.-% |
|---|---|
| Natronlauge 45% | 0,30 |
| Wasser | ad 100,00 |

**Beispiel 6**

| W/O - Emulsion | Gew.-% |
|---|---|
| PEG-30-Dipolyhydroxystearat | 4,00 |
| Caprylsäure/Caprinsäuretriglycerid | 5,00 |
| Octyldodecanol | 5,00 |
| Dicaprylylether | 5,00 |
| Mineralöl | 5,00 |
| Hydriertes Polyisobuten | 5,00 |
| Vitamin-E-Acetat | 0,50 |
| Dioctylbutamidotriazon | 1,00 |
| Bornitrid | 2,00 |
| Aerosil® R972 | 0,50 |
| Konservierung | 0,50 |
| Glycerin | 10,00 |
| $MgSO_4$ | 1,00 |
| Phenylbenzimidazolsulfonsäure | 1,00 |
| Natronlauge 45% | 0,30 |
| Wasser | ad 100,00 |

**Beispiel 7**

| W/O - Emulsion | Gew.-% |
|---|---|
| Cetyldimethiconcopolyol | 5,00 |
| Dimethicon | 5,00 |
| Mineralöl | 2,00 |
| Isohexadekan | 2,00 |
| Butylenglycoldicaprylat/caproat | 8,00 |
| $C_{12-15}$-Alkylbenzoate | 5,00 |
| Bornitrid | 6,00 |
| Methylbenzylidencampher | 4,00 |
| Butylmethoxydibenzoyl methan | 2,00 |
| Titandioxid | 2,00 |
| Konservierung | 0,50 |
| Glycerin | 5,00 |
| NaCl | 1,00 |
| Phenylbenzimidazolsulfonsäure | 2,00 |
| Natronlauge 45% | 0.80 |
| Wasser | ad 100,00 |

**Beispiel 8**

| Spray | Gew.-% |
|---|---|
| Glycerinmonostearat | 4,00 |
| Ceteareth-12 | 1,50 |
| Caprylsäure/Caprinsäuretriglycerid | 2,00 |
| Mineralöl | 5,00 |
| Dioctylbutamidotriazon | 0,50 |
| Bornitrid | 3,00 |
| Octyltriazon | 1,00 |
| Konservierung | 0,50 |
| Glycerin | 10,00 |
| Phenylbenzimidazolsulfonsäure | 4,00 |
| Natronlauge 45% | 1,30 |
| Wasser | ad 100,00 |

**Beispiel 9**

| Spray | Gew.-% |
|---|---|
| Glycerinmonostearat SE | 4,50 |
| Ceteareth-20 | 1,00 |
| Dicaprylylether | 5,00 |
| Cetylstearylisononanoat | 5,00 |
| Dimethicon | 2,00 |
| Bornitrid | 1,00 |
| Methylbenzylidencampher | 2,00 |
| Butylmethoxydibenzoyl methan | 1,00 |
| Konservierung | 0,50 |
| Glycerin | 5,00 |
| Phenylbenzimidazolsulfonsäure | 2,00 |
| Natronlauge 45% | 0,70 |
| Wasser | Ad 100,00 |

**Patentansprüche**

1. Verwendung von Bornitrid zur Stabilisierung von O/W-Emulsionen, die einen Gehalt an Phenylen-1,4-bis-(2-benzimidazyl)-3,3'-5,5'-tetrasulfonsäure bzw. deren Salzen enthalten.

2. Verwendung nach Anspruch 1, **dadurch gekennzeichnet, dass** die Lichtschutzzubereitungen 0,1 bis 20 Gew.-%, vorteilhaft 0,5 bis 10 Gew.-%, ganz besonders bevorzugt 1 bis 5 Gew.-% Bornitrid enthalten.

3. Verwendung nach Anspruch 1 **dadurch gekennzeichnet, daß** die Lichtschutzzubereitungen 0,1 bis 20 Gew.-%, vorteilhaft 0,5 bis 10 Gew.-%, ganz besonders bevorzugt 1 bis 5 Gew.-% an Phenylen-1,4-bis-(2-benzimidazyl)-3,3'-5,5'-tetrasulfonsäure bzw. deren Salzen enthalten.

4. Verwendung nach Anspruch 1, wobei die Emulsionen einen oder mehrere Elektrolyte gelöst enthält, und die Ionenstärke der wäßrigen Phase, in der der oder die Elektrolyte gelöst vorliegen, mindestens 0,0025, vorzugsweise mindestens 0,005, insbesondere mindestens 0,0075 mol/l beträgt.

## Claims

1. Use of boron nitride for stabilizing O/W emulsions which comprise a content of phenylene-1,4-bis(2-benzimidazyl)-3,3'-5,5'-tetrasulphonic acid or salts thereof.

2. Use according to Claim 1, **characterized in that** the light protection preparations comprise 0.1 to 20% by weight, advantageously 0.5 to 10% by weight, very particularly preferably 1 to 5% by weight, of boron nitride.

3. Use according to Claim 1, **characterized in that** the light protection preparations comprise 0.1 to 20% by weight, advantageously 0.5 to 10% by weight, very particularly preferably 1 to 5% by weight, of phenylene-1,4-bis(2-benzimidazyl)-3,3'-5,5'-tetrasulphonic acid or salts thereof.

4. Use according to Claim 1, where the emulsions comprise one or more electrolytes in dissolved form, and the ionic strength of the aqueous phase in which the electrolyte(s) is/are present in dissolved form is at least 0.0025, preferably at least 0.005, in particular at least 0.0075 mol/l.

## Revendications

1. Utilisation de nitrure de bore pour stabiliser des émulsions d'huile dans l'eau contenant de l'acide phénylène-1,4-bis (2-benzimidazyl)-3,3'-5,5'-tétrasulfonique ou, selon le cas, les sels de celui-ci.

2. Utilisation selon la revendication 1, **caractérisée en ce que** les préparations de protection solaire contiennent 0,1 à 20% en poids, avantageusement 0,5 à 10% en poids, de manière particulièrement préférée 1 à 5% en poids de nitrure de bore.

3. Utilisation selon la revendication 1, **caractérisée en ce que** les préparations de protection solaire contiennent 0,1 à 20% en poids, avantageusement 0,5 à 10% en poids, de manière particulière préférée 1 à 5% en poids d'acide phénylène-1,4-bis(2-benzimidazyl)-3,3'-5,5'-tétrasulfonique ou, selon le cas, les sels de celui-ci.

4. Utilisation selon la revendication 1, dans laquelle les émulsions contiennent un ou plusieurs électrolytes dissous et la force ionique de la phase aqueuse, dans laquelle le ou les électrolyte(s) se trouve(nt) sous forme dissoute, s'élève au moins à 0,0025, de préférence au moins à 0,005, en particulier au moins à 0,0075 mole/l.